# EUROPÄISCHE PATENTSCHRIFT

(11) **EP 3 723 725 B1**
(45) Veröffentlichungstag und Bekanntmachung des Hinweises auf die Patenterteilung: **02.02.2022**
(21) Anmeldenummer: 18833180.5
(22) Anmeldetag: 14.12.2018
(51) Int. Cl.: A61K 31/4468, A61K 31/485, A61P 25/04, A61K 9/00, A61K 47/32, A61K 47/34

(54) **MIKRONADELARRAY AUFWEISEND EINEN WIRKSTOFF IN FORM VON SALZEN**
MICRONEEDLE ARRAY HAVING AN ACTIVE INGREDIENT IN THE FORM OF SALTS
MATRICE À MICRO-AIGUILLES PRÉSENTANT UN PRINCIPE ACTIF SOUS FORME DE SELS

(30) Priorität: 14.12.2017 DE 102017130057
(43) Veröffentlichungstag der Anmeldung: 21.10.2020
(73) Patentinhaber: LTS LOHMANN Therapie-Systeme AG, 56626 Andernach (DE)
(72) Erfinder: HENNING, Andreas, 72469 Meßstetten (DE); HILLE, Thomas, 56567 Neuwied (DE); WAUER, Gabriel, 53474 Ahrweiler (DE); SPILGIES, Heiko, 10781 Berlin (DE); PRACHT, Rolf, 56203 Höhr-Grenzhausen (DE)
(74) Vertreter: Simandi, Claus
(86) Internationale Anmeldenummer: PCT/EP2018/085083
(87) Internationale Veröffentlichungsnummer: WO 2019/115815

(56) Entgegenhaltungen:
- WO-A1-2014/191979
- WO-A1-2017/043517
- US-A1- 2005 226 922

## Beschreibung

Die Erfindung betrifft ein Mikronadelarray, wie definiert in den Ansprüchen, insbesondere ein Applikatorsystem, und dessen

Verwendung zur intradermalen Applikation von Wirkstoffen in Form von Salzen, insbesondere Arzneimittel in Form von Salzen, wobei dieser Mikronadelarray zur Hautpenetration an Mensch oder Tier geeignet ist und die Mikronadeln aus einer Formulierung bestehen, die mindestens einen Wirkstoff in Form eines Salzes und mindestens ein biologisch abbaubares Polymer enthalten.

Mikronadelsysteme und Vorrichtungen bei denen Mikronadelarrays zur schmerzfreien intradermalen (bzw. transdermalen) Verabreichung von Wirkstoffen, insbesondere Arzneimitteln verwendet werden, sind im Stand der Technik bekannt. Die transdermale Applikation von Wirkstoffen in Form von Salzen ist zu einem Pflaster bzw. Transdermales therapeutisches System (TTS) in DE 102007041557 B4 der Anmelderin offenbart. Die WO2014/193729 A1 offenbart Mikronadelarrays in einem Applikatorsystem mit Auslösevorrichtung enthaltend sich vollständig auflösende Mikronadeln aus biodegradierbaren Polymeren und Wirkstoffen, wie Fentanylzitrat.

Die Haut besteht aus mehreren Schichten. Die äußerste Hautschicht, das *Stratum Corneum,* weist bekannte Barriereeigenschaften auf, um ein Eindringen von Fremdsubstanzen in den Körper und ein Austreten von Eigensubstanzen aus dem Körper zu verhindern. Das *Stratum Corneum,* das eine komplexe Struktur aus kompaktierten keratotischen Zellresten mit einer Dicke von ungefähr 10-30 Mikrometern ist, bildet hierzu eine wasserdichte Membran zum Schutz des Körpers aus. Diese natürliche Undurchlässigkeit des *Stratum Corneums* verhindert das Verabreichen der meisten pharmazeutischen Mittel und anderer Substanzen durch die Haut im Rahmen einer intradermalen Applikation.

Das Verabreichen verschiedener Substanzen erfolgt daher beispielsweise durch Erzeugen von Mikroporen oder Schnitten im *Stratum Corneum* und Zuführen bzw. Applikation eines Arzneimittels in oder unter das *Stratum Corneum.* Auf diese Weise können zahlreiche Arzneimittel z.B. auch subkutan oder intradermal bzw. intrakutan verabreicht werden.

Im Stand der Technik ist es nach wie vor problematisch, dass ein Wirkstoff in Form zumeist seiner Base appliziert werden kann, diese jedoch instabiler sind als ihre Salzform und folglich Verluste eintreten. Daher besteht ein hohes Bedürfnis, die zu applizierende Wirkstoffe in Form von Salzen in einer geeigneten Arzneiform bereitzustellen.

Daher besteht die objektive Aufgabe der Erfindung darin, eine geeignete Arzneiform zur intradermalen Applikation von Wirkstoffen, wie definiert in den Ansprüchen, in Form von Salzen bereitzustellen.

Die Aufgabe wird durch die vermittelte technische Lehre der Patentansprüche gelöst.

Daher betrifft die Erfindung eine solche mit den Merkmalen des Patentanspruches 1, nämlich ein Mikronadelarray zur Verwendung in der intradermalen Applikation eines Schmerzmittels in Form eines Salzes und zwar Buprenorphinhydrochlorid umfassend eine Vielzahl an Mikronadeln an einem Träger, dadurch gekennzeichnet, dass der Mikronadelarray mittels eines Applikatorsystems appliziert wird, wobei das Applikatorsystem eine Auslösevorrichtung umfasst und die Mikronadeln eine Formulierung umfassen, die mindestens ein Schmerzmittel in Form eines Salzes und zwar Buprenorphinhydrochlorid und mindestens ein wasserlösliches Polymer enthalten.

Besonders vorteilhaft ist, dass die penetrierten Mikronadeln in-situ aufgelöst werden und unmittelbar resorbieren. Damit entfällt die Notwendigkeit, dass die Mikronadeln innen hohl sein müssen oder einen Kanal aufweisen, denn der Wirkstoff in Form von Salzen wird, eingebettet in einer Formulierung, in die Haut gebracht. Im Körper löst sich die Formulierung auf, und der Wirkstoff in Form von Salzen wird freigesetzt. Weiterhin ist an hohlen Mikronadeln nachteilig, dass die Hohlräume durch gerinnendes Blut während der Tragedauer verstopfen können.

Erfindungsgemäß wird Buprenophinhydrochlorid verwendet, das für die intradermale Applikation geeignet ist.

Bei dem Wirkstoff in Form von Salzen handelt es sich um Buprenorphinhydrochlorid. Bevorzugt ist, dass die Salze in einem pharmazeutisch akzeptablen Lösemittel wie zum Beispiel Ethanol löslich sind. Der Begriff "löslich" bedeutet, dass sich 1 Teil des Salzes in 10 - 30 Teilen des Lösemittels löst.

Der Fachmann ist in der Lage zu den Wirkstoffen entsprechende Salze herzustellen.

Der Mikronadelarray kann eine Vielzahl von Mikronadeln aufweisen, um einen Wirkstoffe in Form eines Salzes über die Haut oder in die Haut eines Patienten abgeben zu können, wobei der Mikronadelarray auf die Haut des Patienten aufgebracht wird. Jede der Mikronadeln des Mikronadelarrays weist vorzugsweise einen langgestreckten Schaft mit zwei Enden auf, das eine Ende des Schafts ist die Basis der Mikronadel, mit der die Mikronadel an dem flächigen Träger befestigt oder mit der die Mikronadel in den flächigen Träger integriert ist. Das der Basis gegenüberliegende Ende des Schafts ist vorzugsweise spitz zulaufend ausgestaltet, um ein möglichst leichtes Eindringen der Mikronadel in die Haut zu ermöglichen.

Der erfindungsgemäße Mikronadelarray wie definiert in den Ansprüchen, ist zur Verwendung in der intradermalen Applikation von Buprenorphinhydrochlorid geeignet und umfasst eine Vielzahl an Mikronadeln an einem Träger, wobei die Mikronadeln eine Formulierung umfassen oder aus einer Formulierung bestehen, die mindestens Buprenorphinhydrochlorid und mindestens ein biologisch abbaubares Polymer enthalten.

Bei den biologisch abbaubaren Polymeren handelt es sich um wasserlösliche Polymere, vorzugsweise solche Polymere ausgewählt aus der Gruppe Polyvinylpyrrolidon, Polyvinylalkohole, Cellulose, Dextrane, alpha-Hydroxysäuren, solche wie Milchsäure und/oder Glykolsäure, Polylactide, Polyglykolide, Polylactide-co-glykolide, und Copolymere davon mit Polyethylenglykol, Polyanhydride, Poly(ortho)ester, Polyurethane, Polybuttersäuren, Polyvaleriansäuren, und Polylactid-co-caprolactone.

Im Sinne dieser Erfindung sind ebenfalls solche Polymere wasserlöslich, die sich in Wasser oder Ethanol oder Alkohol-Wassermischungen bei Raumtemperatur bis zu 50 % oder in der Siedehitze, das heißt bei ca. 78°C, bis zu 80 % lösen.

Die Mikronadeln können einen Schaft mit einem runden Querschnitt oder einen nicht runden Querschnitt aufweisen, beispielsweise mit einem dreieckigen, viereckigen oder einem polygonalen Querschnitt. Der Schaft kann einen Durchgang oder mehrere Durchgänge aufweisen, die von der Nadelbasis bis zur Nadelspitze oder annähernd zur Nadelspitze verläuft/verlaufen. Die Mikronadeln können als (Wider-)Haken ausgebildet sein, wobei ein oder mehrere dieser Mikronadeln einen oder mehrere solcher Haken aufweist. Weiterhin können die Mikronadeln schraubenförmig gestaltet und drehbar angeordnet sein und dadurch beim Anwenden einer rotierenden Bewegung das Eindringen in die Haut erleichtern und eine Verankerung in der Haut bewirken (DE 103 53 629 A1), insbesondere in der gewünschten Penetrationstiefe in der Epidermis.

Der Durchmesser einer Mikronadel beträgt üblicherweise zwischen 1 µm und 1000 µm, vorzugsweise zwischen 10 µm und 100 µm. Der Durchmesser eines Durchgangs beträgt üblicherweise zwischen 3 µm und 80 µm und ist für das Durchtreten von vorzugsweise flüssigen Stoffen, Lösungen und Stoffzubereitungen geeignet. Die Länge einer Mikronadel beträgt üblicherweise zwischen 5 µm und 6.000 µm, insbesondere zwischen 100 µm und 700 µm.

Die Mikronadeln sind mit ihrer Basis an einem flächigen Träger befestigt oder in einen flächigen Träger integriert. Die Mikronadeln sind vorzugsweise im Wesentlichen senkrecht zur Fläche des Trägers stehend angeordnet. Die Mikronadeln können regelmäßig oder unregelmäßig angeordnet sein. Eine Anordnung von mehreren Mikronadeln kann Mikronadeln mit unterschiedlichen Querschnittformen, unterschiedlich dimensionierten Durchmessern und/oder unterschiedlichen Längen aufweisen. Die Anordnung kann ebenfalls massive Mikronadeln umfassen, als auch teilmassive Komposite aufweisen.

Die Dichte der Mikronadeln auf einem Träger können 5 - 5.000 Stk/ cm², insbesondere 5 - 1.000 Stk/ cm² betragen.

Der Mikronadelarray kann einen flächigen Träger aufweisen, wobei der Träger im Wesentlichen eine scheibenförmige, plattenförmige oder folienförmige Grundform hat. Der Träger kann eine runde, ovale, dreieckige, viereckige oder polygonale Grundfläche haben. Der Träger kann aus unterschiedlichen Materialien hergestellt sein, beispielsweise aus einem Metall, einem keramischen Werkstoff, einem Halbleiter, einem organischen Material, einem Polymer oder einem Komposit. Als Materialien, die zur Herstellung des Trägers geeignet sind, können vorzugsweise Folien oder bahnförmige Materialien genannt werden, beispielsweise mikroporöse Membranen, vorzugsweise aus Polyethylen (PE) oder Polypropylen (PP), oder Diffusions-Membranen, vorzugsweise aus Ethylen-Vinylacetat-Copolymer (EVA) oder Polyurethan (PUR). Geeignete Materialien zur Herstellung des Trägers können aus der Gruppe ausgewählt sein, die Polyester wie Polyethylenterephthalate (PET), Polycarbonate (PC), Polyetherketone (PAEK) Polyethylennaphthalat (PEN), Polybutylenterephthalate (PBT), Polyurethane (PU), Polystyrole (PS), Polyamide (PA), Polyoxymethylen (POM), Polyolefine wie Polyethylen (PE) und Polypropylen (PP), Polytetrafluorethylen (PTFE), Polyvinylchlorid (PVC), Polyvinylidenchlorid (PVDC), Polylactat (PLA), Polymethylmethacrylat (PMMA) und Kunststoffe auf Cellulose-Basis wie Cellulosehydrat oder Celluloseacetat umfasst. Geeignete Materialien zur Herstellung des Trägers können auch aus der Gruppe der Metalle ausgewählt sein, welche Aluminium, Eisen, Kupfer, Gold, Silber, Platin, Legierungen der vorgenannten Metalle und andere pharmazeutisch annehmbare Metallfolien oder mit Metall bedampfte Folien umfassen. Vorzugsweise ist der Träger aus einem flexiblen Material hergestellt, beispielsweise einem Kunststoff. Ein Träger aus einem flexiblen Material kann sich besser der Hautoberfläche und ihrer Krümmung anpassen als ein Träger aus einem nicht flexiblen Material. Dadurch wird ein besserer Kontakt des Mikronadelarrays mit der Haut erreicht, wodurch die Verlässlichkeit des Mikronadelarrays verbessert wird.

In einer bevorzugten Ausführungsform ist der Mikronadelarray ein ebener oder planarer Mikronadelarray.

In einer weiteren Ausführungsform der Erfindung können Applikatorsysteme zum Auftragen eines Mikronadelarrays unter Druck auf die Haut verwendet werden und führen zu einer schlagartigen Belastung der Haut. Erfindungsgemäß ist das Mikronadelsystem enthaltend einen Mikronadelarray mit einem Applikator ausgestaltet. Solche Applikatoren erlauben vorteilhaft die Aktivierung eines Druckmechanismus zur Penetration des Mikronadelarrays in die Haut bzw. *Stratum Corneum* (siehe z.B. WO2008091602A2, WO2016162449A1).

In einer weiteren Ausführungsform kann das Applikatorsystem enthaltend einen Mikronadelarray mit üblichen funktionellen Gegenständen ausgestaltet sein, welche ein Fixieren auf der Haut erlauben, als auch eine leichte Handhabung zum Druckausüben auf die Haut erlauben und insbesondere mindestens ein Fixiermittel enthalten können.

Im Rahmen dieser Erfindung ist ein Applikatorsystem ein solches System, welches eine Vorrichtung enthält, die bewirkt, dass der Mikronadelarray zur Verabreichung des Buprenorphinhydrochlorids auf der Haut bereitgestellt und der Wirkstoff Buprenorphinhydrochlorid intradermal appliziert wird.

Das Applikatorsystem kann in einer bevorzugten Ausführungsform eine Auslösevorrichtung umfassen, die elektrisch oder mechanisch gesteuert wird. Das Applikatorsystem kann beispielsweise einen Kolben aufweisen, der den Mikronadelarray auf die Haut aufsetzt oder aufbringt, so dass die Mikronadeln in die Haut penetrieren.

Die Auslösevorrichtung kann beispielweise eine Pumpe, eine Spritze oder eine Feder umfassen, so dass ein Kolbenstoß mit hinreichender Energie erfolgen kann. Der Kolben kann beliebiger Form und Natur sein und soll in erster Linie bewerkstelligen, dass der Mikronadelarray von einer ersten Stellung in eine zweite Stellung zur Verabreichung der Wirkstoffe auf der Haut bereitgestellt wird.

Das Applikatorsystem kann weiterhin einen Druckknopf oder Gewinde umfassen.

Gemäß einer weiteren Ausführungsform kann der Mikronadelarray Fixiermittel enthalten, die vorzugsweise mit Hilfe eines haftklebenden Klebestreifens oder Pflasters auf der Haut eines Patienten oder Probanden befestigt werden, auch Nadelpflaster genannt. Als Haftkleber eignen sich hochviskose Stoffe, die nach kurzem leichtem Druck auf der Haut kleben, so genannte druckempfindliche Haftkleber (PSA). Sie besitzen hohe Kohäsions- und Adhäsionskräfte. Zum Einsatz kommen können beispielsweise Haftkleber auf Basis von Poly(meth)acrylaten, auf Basis von Polyisobutylenen oder auf Basis von Silikonen. In einer weiteren Ausführungsform kann das Fixiermittel aus einem Band, elastisches Band, Gummi oder Riemen bestehen. Mithilfe solcher Fixiermittel kann eine sichere Befestigung am Körper erfolgen.

Daher betrifft die Erfindung ebenfalls ein erfindungsgemäßes Applikatorsystem enthaltend einen Mikronadelarray oder ein Mikronadelarray zur intradermalen Applikation, welches Fixiermittel für die Haut aufweist.

Der Begriff "intradermale Applikation (synonym: "intrakutane Applikation") beschreibt erfindungsgemäß die Verabreichung von beliebigen Wirkstoffen über den Mikronadelarray in die Haut und erfordert ein Einstechen oder Penetrieren der Mikronadeln in die Haut.

Daher betrifft die Erfindung ebenfalls ein Verfahren zur intradermalen Applikation, wobei ein Mikronadelarray aufweisend eine Vielzahl an Mikronadeln an einem Träger, insbesondere mittels eines Applikatorsystems appliziert wird, wobei die Mikronadeln aus einer Formulierung bestehen, die mindestens einen Wirkstoff in Form eines Salzes und mindestens ein biologisch abbaubares Polymer enthalten.

Ferner betrifft die Erfindung die Verwendung eines Mikronadelarrays aufweisend eine Vielzahl an Mikronadeln an einem Träger, insbesondere ein entsprechendes Applikatorsystem, wobei die Mikronadeln aus einer Formulierung bestehen, die mindestens einen Wirkstoff in Form eines Salzes und mindestens ein biologisch abbaubares Polymer enthalten, zur intradermalen Applikation.

Nachfolgende Beispiele dienen zur weiteren Erörterung der Erfindung, ohne jedoch die Erfindung auf diese Beispiele zu beschränken.

### Beispiel 1: (nicht erfindungsgemäß)

Man löst in der Siedehitze 100 mg Fentanylcitrat, 6,9 g Resomer R 202 H (Polylactid, PDLLA) und ca. 3 g Ethanol. Noch im heißen Zustand druckt man diese Lösung mit einem 3D-Drucker so auf kreisrunde Polyethylenterephthalat-Folienstücke der Dicke 15µm mit der Fläche 2,5 cm², dass nach Erkalten Nadeln mit einer Höhe von ca. 0,4-0,8 mm entstehen. Diese Nadeln bestehen aus 1,43% Fentanylcitrat und 98,57% Resomer R 202 H und enthalten noch Spuren von Ethanol. Der Drucker wird so eingestellt, dass ca. 20,7 mg, enthaltend 0,3 mg Fentanylcitrat, nach dem Verdunsten des Lösemittels erhalten werden. 0,3 mg Fentanylcitrat entsprechen der täglichen Einstiegsdosis in der transdermalen Schmerztherapie. Die PET Folie wird mit der den Nadeln abgewandten Seite auf ein haftkleberbeschichtetes Gewebe gesetzt, dass das klebende Gewebe das Nadelpflaster überragt. Zur Lagerung werden Nadelpflaster und haftlebendes Gewebe mit einer Tiefziehfolie aus silikonisierter HDPE Folie 175 µm abgcdcckt, um die Polymernadeln zu schützcn. Zur Applikation wird die ticfgczogcnc Folie abgezogen. Das Nadelpflaster wird auf einen Schmerzpatienten geklebt und durch die das Nadelsegment allseitig überragende Kleberschicht fixiert. Die Nadeln aus Fentanylcitrat und Resomer R 202 werden dabei durch die Hornhaut gestoßen. Resomer R 202 zerfällt und gibt hierdurch Fentanylcitrat in das Unterhautgewebe ab, aus dem es verzögert in den Blutkreislauf gelangt.

### Beispiel 2: (nicht erfindungsgemäß)

5 g Fentanylcitrat werden in 95 g Polyvinylalkohol oberhalb der Glasübergangstemperatur der Mischung beider Komponenten per Schmelzextrusion in einen für den jeweiligen 3D Drucker geeigneten homogenen Strang "sog. extruded filament" extrudiert und anschliessend auf Rolle gewickelt. Per 3D-Drucker wird dieser Wirkstoffpolymerstrang aufgeschmolzen und in Form von Nadeln gebracht. Es entstehen Nadeln mit einer Höhe von 0,5-1,0 mm. Hierbei wird die Raumtemperatur und Feuchte möglichst geringgehalten, um den Aushärteprozess möglichst rasch zu gestalten und eine Wasseraufnahme des hygroskopischen Polymers zu verhindern. Die Folie wird mit der den Nadeln abgewandten Seite auf eine haftkleberbeschichtete Folie gesetzt, dass die klebende Folie als "Überpflaster" das Nadelpflaster überragt. Zur Lagerung werden Nadelpflaster und haftlebendes Gewebe mit einer Tiefziehfolie aus silikonisierter HDPE Folie 175 µm abgedeckt, um die Polymernadeln zu schützen. Zur Applikation wird die tiefgezogene Folie abgezogen. Das Nadelpflaster wird auf einen Schmerzpatienten geklebt und durch die das Nadelsegment allseitig überragende Kleberschicht fixiert. Die Nadeln aus Fentanylcitrat und Polyvinylalkohol werden dabei durch die Hornhaut gestoßen. Polyvinylalkohol löst sich auf und gibt hierdurch Fentanylcitrat unterhalb des *Stratum Corneums* ab, aus dem es in den Blutkreislauf gelangt.

### Beispiel 3: (nicht erfindungsgemäß)

5 g Fentanylcitrat werden in 95 g Polyvinylalkohol auf eine Temperatur oberhalb der Schmelztemperatur der Mischung erwärmt und per Injection moulding Verfahren die Schmelze in Form von Nadeln gebracht. Es entstehen Nadeln mit einer Höhe von 0,5-1,0 mm. Hierbei wird die Raumtemperatur und Feuchte möglichst geringgehalten, um den Aushärteprozess möglichst rasch zu gestalten und eine Wasseraufnahme des hygroskopischen Polymers zu verhindern. Die Folie wird mit der den Nadeln abgewandten Seite auf eine haftkleberbeschichtete Folie gesetzt, dass die klebende Folie als "Überpflaster" das Nadelpflaster überragt. Zur Lagerung werden Nadelpflaster und haftlebendes Gewebe mit einer Tiefziehfolie aus silikonisierter HDPE Folie 175 µm abgedeckt, um die Polymernadeln zu schützen. Zur Applikation wird die tiefgezogene Folie abgezogen. Das Nadelpflaster wird auf einen Schmerzpatienten geklebt und durch die das Nadelsegment allseitig überragende Kleberschicht fixiert. Die Nadeln aus Fentanylcitrat und Polyvinylalkohol werden dabei durch die Hornhaut gestoßen. Polyvinylalkohol löst sich auf und gibt hierdurch Fentanylcitrat unterhalb des *Stratum Corneums* ab, aus dem es in den Blutkreislauf gelangt.

### Beispiel 4:

7.5 g Buprenorphin-HCL werden in 92.5 g Polyvinylalkohol auf eine Temperatur oberhalb der Schmelztemperatur der Mischung erwärmt, per Zweischneckenextruder extrudiert und der resultierende Strang per 3D Druckverfahren in Form von Nadeln auf eine für Wirkstoffe undurchlässige Folie gedruckt, so dass Nadeln mit einer Höhe von 0,5-1,0 mm entstehen. Hierbei wird die Raumtemperatur und Feuchte möglichst geringgehalten, um den Aushärteprozess möglichst rasch zu gestalten und eine Wasseraufnahme des hygroskopischen Polymers zu verhindern. Die Folie wird mit der den Nadeln abgewandten Seite auf eine haftkleberbeschichtete Folie gesetzt, dass die klebende Folie als "Überpflaster" das Nadelpflaster überragt. Zur Lagerung werden Nadelpflaster und haftlebendes Gewebe mit einer Tiefziehfolie aus silikonisierter HDPE Folie 175 µm abgedeckt, um die Polymernadeln zu schützen. Zur Applikation wird die tiefgezogene Folie abgezogen. Das Nadelpflaster wird auf einen Schmerzpatienten geklebt und durch die das Nadelsegment allseitig überragende Kleberschicht fixiert. Die Nadeln aus Buprenorphin-HCL und Polyvinylalkohol werden dabei durch die Hornhaut gestoßen. Polyvinylalkohol löst sich auf und gibt hierdurch Buprenorphin-HCL unterhalb des *Stratum Corneums* ab, aus dem es in den Blutkreislauf gelangt.

### Beispiel 5:

5 g Buprenorphin-HCL werden in 25 g einer 30% -igen ethanolischen Lösung aus Polyvinlypyrrolidon K30 gelöst und in eine Matritze enthaltend Negativvertiefungen für ein Nadelarray von z.B. 64 Nadeln mit einer Länge von ca. 0,8mm und einer Breite von ca. 0,2mm bei einem Nadelabstand von ca. 0,3 mm dosiert. Darüber befindlich befindet sich eine Aussparung für eine Arrayplatte mit einer Höhe von ca. 0,2 mm und Fläche von 1 cm². Nachdem das Lösemittel herausgetrocknet wurde, wird eine wirkstofffreie Basisplatte bestehend aus einer 30% -igen methanolischen Lösung aus PVPVA64 über die Nadeln dosiert, um eine leicht biegsame Platte zu erzeugen, die gleichzeitig die Nadeln als Einheit verbindet. Die Folie wird mit der den Nadeln abgewandten Seite auf eine haftkleberbeschichtete Folie gesetzt, dass die klebende Folie als "Überpflaster" das Nadelpflaster überragt. Zur Lagerung werden Nadelpflaster und haftlebendes Gewebe mit einer Tiefziehfolie aus silikonisierter HDPE Folie 175 µm abgedeckt, um die Polymernadeln zu schützen. Zur Applikation wird die tiefgezogene Folie abgezogen. Das Nadelpflaster wird auf einen Schmerzpatienten geklebt und durch die das Nadelsegment allseitig überragende Kleberschicht fixiert. Die Nadeln aus Buprenorphin-HCL und Polyvinylpyrrolidon werden dabei durch die Hornhaut gestoßen. PVP K30 löst sich auf und gibt hierdurch Buprenorphin-HCL unterhalb des *Stratum Corneums* ab, aus dem es in den Blutkreislauf gelangt.

## Patentansprüche

1. Mikronadelarray zur Verwendung in der intradermalen Applikation eines Schmerzmittels in Form eines Salzes und zwar Buprenorphinhydrochlorid umfassend eine Vielzahl an Mikronadeln an einem Träger, **dadurch gekennzeichnet, dass** der Mikronadelarray mittels eines Applikatorsystems appliziert wird, wobei das Applikatorsystem eine Auslösevorrichtung umfasst und die Mikronadeln eine Formulierung umfassen, die mindestens ein Schmerzmittel in Form eines Salzes und zwar Buprenorphinhydrochlorid und mindestens ein wasserlösliches Polymer enthalten.

2. Mikronadelarray zur Verwendung in der intradermalen Applikation eines Schmerzmittels in Form eines Salzes nach Anspruch 1, **dadurch gekennzeichnet, dass** das Applikatorsystem eine Auslösevorrichtung aufweisend einen Kolben, Druckknopf oder Gewinde umfasst.

3. Mikronadelarray zur Verwendung in der intradermalen Applikation nach einem der vorstehenden Ansprüche, **dadurch gekennzeichnet, dass** das wasserlösliche Polymer aus der Gruppe Polyvinylpyrrolidon, Polyvinylalkohole, Cellulose, Dextrane, alpha-Hydroxysäuren, solche wie Milchsäure und/oder Glykolsäure, Polylactide, Polyglykolide, Polylactid-co-Glykolide ist, und Copolymere davon mit Polyethylenglykole, Polyanhydride, Poly(ortho)ester, Polyurethane, Polybuttersäuren, Polyvaleriansäuren, und Polylactid-co-caprolactone ausgewählt ist.

4. Mikronadelarray zur Verwendung in der intradermalen Applikation nach einem der vorstehenden Ansprüche, **dadurch gekennzeichnet, dass** der Mikronadelarray planar ist.

5. Mikronadelarray zur Verwendung in der intradermalen Applikation nach einem der vorstehenden Ansprüche, **dadurch gekennzeichnet, dass** die Dichte der Mikronadeln auf einem Träger 5 - 5.000 Stk/ cm² beträgt.

6. Mikronadelarray zur Verwendung nach einem der vorstehenden Ansprüche, **dadurch gekennzeichnet, dass** der Mikronadelarray Fixiermittel aufweist, insbesondere ausgewählt aus der Gruppe Klebestreifen, Pflaster, Band, elastisches Band, Gummi oder Riemen.

7. Applikatorsystem enthaltend einen Mikronadelarray zur Verwendung in der intradermalen Applikation nach einem der vorstehenden Ansprüche, **dadurch gekennzeichnet, dass** das Applikatorsystem einen Auslösemechanismus umfasst.

8. Applikatorsystem enthaltend einen Mikronadelarray zur Verwendung in der intradermalen Applikation nach Anspruch 7, **dadurch gekennzeichnet, dass** der Auslösemechanismus einen Kolben, Druckknopf oder Gewinde umfasst.

9. Applikatorsystem enthaltend einen Mikronadelarray zur Verwendung in der intradermalen Applikation nach Anspruch 7, **dadurch gekennzeichnet, dass** der Auslösemechanismus elektrisch oder mechanisch gesteuert wird.

## Claims

1. A microneedle array for use with the intradermal delivery of an analgesic in the form of a salt, namely buprenorphine hydrochloride, comprising a plurality of microneedles on a carrier, **characterized in that** the microneedle array is applied by way of an applicator system, the applicator system comprising a trigger device, and the microneedles comprising a formulation containing at least one analgesic in the form of a salt, namely buprenorphine hydrochloride, and at least one water-soluble polymer.

2. The microneedle array for use with the intradermal delivery of an analgesic in the form of a salt according to claim 1, **characterized in that** the applicator system comprises a trigger device comprising a plunger, a push button or a thread.

3. A microneedle array for use with intradermal delivery according to any one of the preceding claims, **characterized in that** the water-soluble polymer is selected from the group consisting of polyvinylpyrrolidone, polyvinyl alcohols, cellulose, dextrans, alpha-hydroxy acids, such as lactic acid and/or glycolic acid, polylactides, polyglycolides, polylactide-co-glycolides, and copolymers thereof with polyethylene glycols, polyanhydrides, poly(ortho)esters, polyurethanes, polybutyric acids, polyvaleric acids, and polylactide-co-caprolactones.

4. A microneedle array for use with intradermal delivery according to any one of the preceding claims, **characterized in that** the microneedle array is planar.

5. A microneedle array for use with intradermal delivery according to any one of the preceding claims, **characterized in that** the density of the microneedles on a carrier is 5 to 5,000 pieces/cm².

6. A microneedle array for use according to any one of the preceding claims, **characterized in that** the microneedle array comprises fixation means, in particular selected from the group consisting of adhesive strip, patch, band, elastic band, rubber or strap.

7. The applicator system comprising a microneedle array for use with intradermal delivery according to any one of the preceding claims, **characterized in that** the applicator system comprises a trigger mechanism.

8. The applicator system comprising a microneedle array for use with intradermal delivery according to claim 7, **characterized in that** the trigger mechanism comprises a plunger, a push button, or a thread.

9. The applicator system comprising a microneedle array for use with intradermal delivery according to claim 7, **characterized in that** the trigger mechanism is electrically or mechanically controlled.

## Revendications

1. Matrice de micro-aiguilles destinée à l'utilisation dans l'application intradermique d'un analgésique sous la forme d'un sel, à savoir le chlorhydrate de buprénorphine, comprenant une pluralité de micro-aiguilles sur un support, **caractérisé en ce que** le réseau de micro-aiguilles est appliqué au moyen d'un système applicateur, le système applicateur comprenant un dispositif de déclenchement et les micro-aiguilles comprenant une formulation contenant au moins un analgésique sous la forme d'un sel, à savoir le chlorhydrate de buprénorphine, et au moins un polymère hydrosoluble.

2. Matrice de micro-aiguilles destinée à l'utilisation dans l'application intradermique d'un analgésique sous la forme d'un sel selon la revendication 1, **caractérisée en ce que** le système applicateur comprend un dispositif de déclenchement comprenant un plongeur, un bouton-poussoir ou un fil.

3. Matrice de micro-aiguilles destinée à l'utilisation dans l'application intradermique selon l'une quelconque des revendications précédentes, **caractérisée en ce que** le polymère hydrosoluble est choisi dans le groupe constitué par la polyvinylpyrrolidone, les alcools polyvinyliques, la cellulose, les dextranes, les alpha-hydroxyacides, comme l'acide lactique et/ou l'acide glycolique, les polylactides, les polyglycolides, les polylactide-co-glycolides et leurs copolymères avec les polyéthylèneglycols, les polyanhydrides, les poly(ortho)esters, les polyuréthanes, les acides polybutyriques, les acides polyvalents et les polylactide-co-caprolactones.

4. Matrice de micro-aiguilles destinée à l'utilisation dans l'application intradermique selon l'une quelconque des revendications précédentes, **caractérisée en ce que** la matrice de micro-aiguilles est plane.

5. Matrice de micro-aiguilles destinée à l'utilisation dans l'application intradermique selon l'une quelconque des revendications précédentes, **caractérisée en ce que** la densité des micro-aiguilles sur un support est de 5 à 5 000 pcs/cm.²

6. Matrice de micro-aiguilles destinée à l'utilisation selon l'une quelconque des revendications précédentes, **caractérisée en ce que** la matrice de micro-aiguilles comprend des moyens de fixation, notamment choisis dans le groupe constitué par les bandes adhésives, les sparadraps, le ruban adhésif, le ruban élastique, le caoutchouc ou les sangles.

7. Système applicateur contenant une matrice de micro-aiguilles pour une utilisation en application intradermique selon l'une quelconque des revendications précédentes, **caractérisé en ce que** le système applicateur comprend un mécanisme de déclenchement.

8. Système applicateur contenant une matrice de micro-aiguilles pour une utilisation en application intradermique selon la revendication 7, **caractérisé en ce que** le mécanisme de déclenchement comprend un plongeur, un bouton poussoir ou un fil.

9. Système applicateur contenant une matrice de micro-aiguilles pour une utilisation en application intradermique selon la revendication 7, **caractérisé en ce que** le mécanisme de déclenchement est contrôlé électriquement ou mécaniquement.
